# EUROPÄISCHE PATENTSCHRIFT

(11) **EP 0 620 233 B1**
(45) Veröffentlichungstag und Bekanntmachung des Hinweises auf die Patenterteilung: **27.12.1996**
(21) Anmeldenummer: 94104818.3
(22) Anmeldetag: 26.03.1994
(51) Int. Cl.: C08F 220/06, C08F 2/14, C08F 287/00

(54) **Poly(meth)acrylsäure-Dispersion**
Dispersion of poly(meth)acrylic acid
Dispersion de poly-acide acrylique au méthacrylique

(30) Priorität: 10.04.1993 DE 4311916
(43) Veröffentlichungstag der Anmeldung: 19.10.1994
(73) Patentinhaber: Th. Goldschmidt AG, D-45127 Essen (DE)
(72) Erfinder: Fock, Jürgen, Dr., D-40470 Düsseldorf (DE); Koerner, Götz, Dr., D-45259 Essen (DE); Reichert, Karl-Heinz, Prof. Dr., D-12623 Berlin (DE); Fengler, Stephan, D-12309 Berlin (DE); Smolin, Roland, D-10553 Berlin (DE)

(56) Entgegenhaltungen:
- EP-A- 0 522 467
- US-A- 3 284 393
- US-A- 3 996 180
- JOURNAL DE CHIMIE PHYSIQUE, Bd.78, Nr.3, 1981 Seiten 279 - 284 LEONG ET AL. 'POLYMERISATION D'ACRYLAMIDE A L'INTERIEUR DE MICELLES MACROMOLECULAIRES'

## Beschreibung

Die Erfindung betrifft eine Poly(meth)acrylsäure-Dispersion mit einem organischen Lösungsmittel als äußerer Phase, wobei die disperse Phase Teilchen einer mittleren Teilchengröße von etwa 20 bis 300 nm bei geringer Polydispersität aufweist.

Es ist aus dem Stand der Technik bekannt, Acrylgruppen enthaltende Monomere in Form einer Wasser-in-Öl-Emulsion zu polymerisieren. Dabei werden Dispersionen erhalten, deren Teilchen im allgemeinen größer als 300 nm sind. Die Teilchen weisen dabei ein relativ breites Spektrum der Teilchengröße auf.

Aus dem umfangreichen Stand der Technik wird stellvertretend auf folgende Veröffentlichungen hingewiesen:

Im "Journal de chimie physique" 1981, 78, Nr. 3, berichten Y.S. Leong, G. Riess und F. Candau über die Photopolymerisation von Acrylamid in einer Wasser-in-Öl-Emulsion unter Verwendung eines öllöslichen Radikalbildners. Die Reaktion wird dabei in Gegenwart eines Polystyrol-Polyoxyalkylen-Blockcopolymeren als Stabilisator und Propanol in Toluol-Wasser-Mischungen durchgeführt. Die erhaltenen Latices sind transparent und bestehen, wie sich aus Dialyseversuchen ergibt, aus in Wasser gequollenen micellaren Lösungen. Das Molekulargewicht des erhaltenen Polyacrylamids liegt im Bereich von 50 000 bis 130 000.

Aus "Colloid Polym. Sci." 264 (7), 616, ist es bekannt, Polyvinylacetat und Polymethylmethacrylat in Form von Teilchen mit einem Durchmesser von 100 bis 300 nm durch Dispersionspolymerisation in nichtwäßrigem Medium in Gegenwart von Poly(Styrol-b-[Ethylen-co-Propylen]) herzustellen. Der Teilchendurchmesser sinkt dabei mit zunehmender Konzentration des Blockcopolymeren.

Aus der GB-PS 2 140 433 ist es bekannt, stabile Wasser-in-Öl-Emulsionen mit hohem Polymergehalt und niedriger Viskosität durch Zugabe der wäßrigen Lösung eines wasserlöslichen Monomeren, wie z. B. Acrylsäure, zu einer inerten hydrophoben organischen Flüssigkeit durch radikalische Polymerisation zu erhalten.

Die US-PS 3 284 393 betrifft ein Verfahren, bei dem wasserlösliche Monomere, wie Acrylsäure, Methacrylsäure, Acrylamid und andere Monomere, in wäßriger Lösung in einem nicht mit Wasser mischbaren Öl in Gegenwart eines niedermolekularen Emulgators, wie Sorbitanmonooleat, und eines Puffers emulgiert werden. Die Polymerisation wird mit öllöslichen Radikalinitiatoren durchgeführt. Es werden Polymerteilchen eines Durchmessers von etwa 100 nm bei einer Reaktionstemperatur von 60 °C im Verlauf einer Reaktionsdauer von 48 Stunden erhalten.

Die vorliegende Erfindung befaßt sich mit dem technischen Problem, Poly(meth)acrylsäure-Dispersionen mit einem organischen Lösungsmittel als äußere Phase herzustellen, wobei angestrebt wird, daß die disperse Phase Teilchen mit einer wesentlich niedrigen Teilchengröße von etwa 20 bis 300 nm aufweist und wobei zusätzlich die Polydispersität der Teilchen, d. h. ihre Streuung in bezug auf die Teilchengröße, möglichst gering sein soll. Das Verfahren soll dabei in einfacher Weise, in möglichst quantitativen Ausbeuten und innerhalb wirtschaftlich vertretbaren Polymerisationszeiten ablaufen. Die erhaltenen Dispersionen sollen eine hohe Stabilität aufweisen.

Entsprechend der vorliegenden Erfindung sind derartige Poly(meth)acrylsäure-Dispersionen mit einem organischen Lösungsmittel als äußerer Phase, wobei die disperse Phase Teilchen einer mittleren Teilchengröße von etwa 20 bis 300 nm bei geringer Polydispersität aufweist, erhältlich durch radikalische Polymerisation von (Meth)acrylsäure und gegebenenfalls bis zu 5 Gew.-% Comonomeren, welche eine Vernetzung der Poly(meth)acrylsäure bewirken, in einem unpolaren organischen Lösungsmittel in Gegenwart von Stabilisatoren, Costabilisatoren und Initiatoren, wobei
(1) der Stabilisator ein Blockcopolymerisat ist, welches aus mindestens einem Block A und mindestens einem Block B besteht, wobei der Block A durch Polymerisation von Vinylgruppen enthaltenden Monomeren gebildet ist und der Block B ein Polyoxyalkylenblock ist,
(2) das Gewichtsverhältnis von Stabilisator zur Summe von (Meth)acrylsäure und gegebenenfalls enthaltenen Comonomeren etwa 1 : 3 bis 1 : 100 beträgt,
(3) der Costabilisator Wasser oder ein Alkandiol mit bis zu 3 Kohlenstoffatomen ist, und
(4) das Gewichtsverhältnis von Costabilisator zur Summe von (Meth)acrylsäure und gegebenenfalls enthaltenen Comonomeren ≥ 1 : 50 und höchstens 1 : 1 beträgt, und
(5) das Gewichtsverhältnis von äußerer zu innerer Phase etwa 100 : 5 bis 100 : 70 beträgt.

Die verwendete Schreibweise Poly(meth)acrylsäure soll dabei bedeuten, daß das Polymerisat aus Acrylsäure und/oder Methacrylsäure als Monomere aufgebaut ist. Das gegebenenfalls für die Copolymerisation eingesetzte vernetzende Comonomer ist eine Verbindung mit zwei olefinischen Doppelbindungen, insbesondere Divinylbenzol, Ethylenglykol-, Diethylenglykol- oder Butandiol-1,4-di(meth)acrylat.

In einer weiteren Ausgestaltung der Erfindung kann die erhaltene Poly(meth)acrylsäure mit einem Diglycidylether, vorzugsweise mit Butylenglykoldiglycidylether, nachträglich vernetzt werden.

Die erfindungsgemäßen Poly(meth)acrylsäure-Dispersionen sind milchig trübe bis transparente, bläulich bis rötlich schimmernde Dispersionen, die auch bei längerem Stehen nicht koagulieren, sondern hohe Stabilität aufweisen und deren Farbe bei Erwärmung bis zur Siedetemperatur der Ölphase reversiblen Veränderungen von blau bis rot unterliegt.

Von besonderer Bedeutung ist dabei das als Stabilisator benutzte Blockcopolymerisat. Dieses besteht aus mindestens einem Block A, der durch Polymerisation von Vinylgruppen enthaltenden Monomeren, wie insbesondere Styrol, Alkylstyrol oder einem Methacrylat gebildet ist, und mindestens einem Block B, der die Bedeutung eines Polyoxyalkylenblockes hat. Dieses Blockcopolymerisat entspricht vorzugsweise dem Typ A-B oder A-B-A. Ein Blockcopolymerisat des Typs A-(B-A-)ₙA (n bedeutet dabei eine Zahl > 0) kann zwar verwendet werden, bringt jedoch gegenüber dem Blockcopolymerisat des Typs A-B oder A-B-A keinen Vorteil.

Die Molmasse dieses in der erfindungsgemäßen Dispersion enthaltenen Stabilisators beträgt etwa 1000 bis 20 000. Bevorzugt ist das Verhältnis der anteiligen Molmassen der Blöcke A und B etwa 0,5 : 1 bis 9 : 1.

Der Polyoxyalkylenblock B besteht vorzugsweise aus mindestens 70 Mol-% Oxyethyleneinheiten, wobei die restlichen Oxyalkyleneinheiten solche mit einer größeren Kohlenstoffzahl als 2 bedeuten können. Beispielsweise kann der Block B aus 70 Mol-% Oxyethyleneinheiten und 30 Mol-% Oxypropyleneinheiten bestehen. Vorzugsweise enthält der Block B jedoch ausschließlich Oxyethyleneinheiten.

Besonders bevorzugt sind Blockcopolymerisate des Typs A-B, wobei der Block A ein Polystyrolblock und der Block B ein Polyoxyethylenblock ist und die Molmasse des Stabilisators etwa 1000 bis 6000 beträgt.

Als Stabilisatoren besonders geeignete Blockcopolymerisate sind in der DE-PS 41 34 967 beschrieben. Dabei sind Blockmischpolymerisate des Typs A-B dadurch erhältlich, daß man zuerst Vinylgruppen aufweisende Monomere in an sich bekannter Weise in Gegenwart ausreichender Mengen eines Initiators und der gewünschten Kettenlänge entsprechenden Mengen eines Kettenreglers, welcher neben einer Mercaptogruppe noch eine weitere, mindestens einen aktiven Wasserstoffrest aufweisende funktionelle Gruppe trägt, bei Temperaturen von 60 bis 150°C radikalisch polymerisiert, das erhaltene Polymerisat in dem Falle, daß die funktionelle Gruppe mit einem aktiven Wasserstoffrest eine OH- oder COOH-Gruppe ist, mit Alkalihydroxid oder Alkalialkoholat unter Entferung von Wasser bzw. Alkohol umsetzt, und an das derart modifizierte Polymerisat Alkylenoxid bis zum Erreichen des gewünschten Molgewichts des Blocks B bei Temperaturen von 20 bis 180°C addiert. Beispiel eines für die Herstellung besonders geeigneten Kettenreglers, welcher neben einer Mercaptogruppe noch eine weitere, mindestens einen aktiven Wasserstoffrest aufweisende Gruppe trägt, ist 1-Mercaptoethan-2-ol. Diese Blockcopolymerisate sind hochwirksame Polymertenside.

Ein weiteres wesentliches Merkmal der erfindungsgemäßen Poly(meth)acrylsäure-Dispersion besteht darin, daß bei ihrer Herstellung das Gewichtsverhältnis des vorgenannten Stabilisators zur Summe von (Meth)acrylsäure und gegebenenfalls enthaltenen Comonomeren etwa 1 : 3 bis 1 : 100, vorzugsweise etwa 1 : 10 bis 1 : 20 beträgt.

Das dritte Merkmal der erfindungsgemäßen Poly(meth)acrylsäure-Dispersion besteht darin, daß bei ihrer Herstellung ein Costabilisator verwendet wird, welcher Wasser oder ein Alkandiol mit bis zu 3 Kohlenstoffatomen ist. Beispiele solcher Alkandiole sind 1,2-Ethandiol und 1,3-Propandiol. Jedoch ist Wasser als Costabilisator bevorzugt.

Gemäß einem weiteren Merkmal der Erfindung ist es erfindungswesentlich, ein bestimmtes Gewichtsverhältnis des Costabilisators, insbesondere Wasser, zu Monomeren, also Acrylsäure und/oder Methacrylsäure und gegebenenfalls enthaltenen Comonomeren einzuhalten. Das Gewichtsverhältnis des Costabilisators zur Summe von (Meth)acrylsäure und gegebenenfalls enthaltenen Comonomeren soll mindestens ≥ 1 : 50 und höchstens 1 : 1 betragen. Vorzugsweise wird als Costabilisator Wasser in einem Gewichtsverhältnis von Wasser zu (Meth)acrylsäure und gegebenenfalls enthaltenen Comonomeren von ≥ 1 : 20 bis ≥ 1 : 5 eingesetzt. Durch die Einhaltung dieses Gewichtsverhältnisses von Costabilisator zu Monomeren wird gewährleistet, daß die erfindungsgemäße Poly(meth)acrylsäure-Dispersion Teilchen der gewünschten Teilchengröße von 20 bis 300 nm aufweist.

Schließlich soll das Gewichtsverhältnis von äußerer zu innerer Phase etwa 100 : 5 bis 100 : 70 betragen.

Die äußere Phase der erfindungsgemäßen Poly(meth)acrylsäure-Dispersion wird durch ein unpolares organisches Lösungsmittel gebildet. Diese Aussage ist natürlich so zu verstehen, daß in dem organischen Lösungsmittel zusätzlich der Costabilisator und der Stabilisator zumindest in den wesentlichen Mengen enthalten sind. Als unpolare organische Lösungsmittel eignen sich insbesondere und bevorzugt aromatische Lösungsmittel oder Lösungsmittelgemische und insbesondere solche mit einem Siedepunkt ≤ 150°C. Besonders bevorzugt bildet Toluol die äußere Phase.

Die Herstellung der erfindungsgemäßen Poly(meth)acrylsäure-Dispersion kann unter Einhaltung der im Vorstehenden genannten Bedingungen in bezug auf die Auswahl des Stabilisators und des Costabilisators und in bezug auf die Mengenverhältnisse von Stabilisator und Costabilisator zur Summe von (Meth)acrylsäure und gegebenenfalls enthaltenen Comonomeren in der Weise vorzugsweise erfolgen, daß man
(1) den Stabilisator und die (Meth)acrylsäure, gegebenenfalls zusammen mit den einzusetzenden Comonomeren, in dem Lösungsmittel löst,
(2) in dieser Lösung den Costabilisator löst oder verteilt,
(3) die Lösung mit Stickstoff begast,
(4) den Initiator zugibt und dann
(5) die Polymerisation bei gegebenenfalls erhöhter Temperatur ablaufen läßt.

Im Falle der Verwendung eines vernetzenden Comonomeren kann man dieses auch nach dem Ablauf der Polymerisation zugeben und dann die Vernetzungsreaktion bei erhöhten Temperaturen von 60 bis 100°C durchführen.

Bei diesem Verfahren kann sich, insbesondere wenn man die maximal zulässigen Mengen Stabilisator, insbesondere Wasser, zu (Meth)acrylsäure verwendet, zunächst eine inhomogene Emulsion bilden, die sich jedoch im Laufe der fortschreitenden Polymerisation in die gewünschte milchig trübe bis transparente, farbig irisierende Dispersion umwandelt.

Als Initiatoren können die an sich bekannten öllöslichen Polymerisationsinitiatoren, wie z.B. Azobis(2,4-Dimethyl-valeronitril), verwendet werden.

Dieses generelle Verfahren der Herstellung kann dadurch abgeändert werden, daß man zunächst nur eine Teilmenge der (Meth)acrylsäure und gegebenenfalls der Comonomeren in Gegenwart der ganzen Menge Costabilisator oder ebenfalls nur einer Teilmenge hiervon polymerisiert und während oder nach der Polymerisation dieser ersten Teilmenge die restliche Menge der (Meth)acrylsäure und gegebenenfalls der Comonomeren, gegebenenfalls mit der restlichen Menge des Costabilisators, gegebenenfalls portionsweise in zeitlichem Abstand, zugibt und die Polymerisation zu Ende führt. Dabei kann man jeweils bei den einzelnen Polymerisationsschritten Stabilisator und gegebenenfalls Costabilisator innerhalb der angegebenen Grenzen nachdosieren. Bei dieser abgeänderten Verfahrensweise wird eine besonders enge Verteilung der Teilchengröße innerhalb der Dispersion erzielt.

Die nach diesem Verfahren durchgeführte Polymerisation erfolgt mit hoher Geschwindigkeit, die während der Reaktion autokatalytisch zunimmt.

Man erhält entsprechend der Erfindung Poly(meth)acrylsäure-Dispersionen einer Teilchengröße von etwa 20 bis 300 nm, insbesondere 20 bis 200 nm Durchmesser, wobei die Teilchengrößenverteilung innerhalb enger Grenzen liegt.

Die erhaltenen erfindungsgemäßen Poly(meth)acrylsäure-Dispersionen zeigen bei Erwärmung einen reversiblen Farbumschlag innerhalb eines Temperaturintervalls von Raumtemperatur bis zur Siedetemperatur der Ölphase. Bei der Abkühlung der Dispersion tritt wieder die ursprüngliche Färbung auf, so daß diese Dispersionen als Temperaturindikatoren verwendet werden können.

Die erfindungsgemäßen Dispersionen können als optische Filtermedien eingesetzt werden. Die erfindungsgemäßen Dispersionen sind aber insbesondere im Hinblick auf ihre niedrige Teilchengröße außerordentlich reaktive Modifizierungsmittel für Produkte, die zur Reaktion mit Carboxylgruppen geeignet sind.

Es ist natürlich dem Fachmann ohne weiteres möglich, aus den Dispersionen die Poly(meth)acrylsäure-Teilchen abzuscheiden, von der organischen Phase abzutrennen und die erhaltenen Polymerteilchen der weiteren Verwendung zuzuführen. Besonders bevorzugte Anwendungsbereiche sind die Verwendung der Polymerteilchen als Superabsorbentien, insbesondere in kosmetischen oder hygienischen flüssigkeitsabsorbierenden Materialien, wie z.B. Höschenwindeln, oder als Träger für Wirkstoffdepots, insbesondere im Bereich der Galenik. Die Teilchen können ferner Trägermaterialien für Katalysatoren bilden. Sie wirken in wäßrigen Lösungen verdickend, wobei das Ausmaß der Verdickung pH-abhängig ist. Sie eignen sich ferner als Chromatographieträgermaterialien.

In den folgenden Beispielen wird die Herstellung erfindungsgemäßer feinteiliger Polyacrylsäure-Dispersionen beschrieben.

### Beispiel 1

In einem Reaktor, der mit einem Rührer hoher Drehzahl ausgerüstet ist, werden 15 g Acrylsäure und 4 g eines Polystyrol-Block-Polyoxyethylen-Copolymeren, wobei der Polystyrolblock eine Molmasse von 3000 und der Polyoxyethylenblock eine Molmasse von 1000 aufweist, in 150 g Toluol gelöst und unter Stickstoff auf 50°C erwärmt. Es werden bei einer Rührgeschwindigkeit von 400 Umdrehungen pro Minute 3 g Wasser zugegeben, wobei weiter Stickstoff durch die Lösung geleitet wird. Die Polymerisation wird durch Zugabe von 0,2 g Azobis(2,4-Dimethyl-valeronitril) gestartet. Nach einer Reaktionsdauer von 20 Minuten werden noch einmal 15 g Acrylsäure und 3 g Wasser zugegeben. Nach einer Gesamtreaktionsdauer von ca. 40 Minuten ist die Reaktion beendet.

Nach dem Abkühlen auf ca. 20°C wird eine transparente, blau irisierende Dispersion erhalten. Der Restmonomerengehalt beträgt ≤ 0,5 Gew.-%, bezogen auf die Dispersion. Die Teilchengröße wird durch Lichtstreuung ermittelt und beträgt ca. 110 nm.

Bei Verwendung eines Blockcopolymeren auf der Basis eines A-B-Copolymeren aus einem Polystyrol- und einem Polyoxyethylenblock, wobei der Polystyrolblock eine Molmasse von 1000 und der Polyoxyethylenblock ebenfalls eine Molmasse von 1000 aufweist, werden Teilchen mit einem Durchmesser von ca. 160 nm erhalten.

Bei Verwendung eines Blockcopolymeren mit der Molmasse des Polystyrolblockes von 3000 und Polyoxyethylenblockes von 3000 werden Teilchen mit einem Durchmesser von ca. 70 nm erhalten.

### Beispiel 2

In einen Reaktor, der mit einem Rührer hoher Drehzahl ausgerüstet ist, werden 20 g Acrylsäure und 1,1 g eines Polystyrol-Block-Polyoxyethylen-Copolymeren, wobei der Polystyrolblock eine Molmasse von 3000 und der Polyoxyethylenblock eine Molmasse von 1000 aufweist, in 150 g Toluol gelöst und unter Stickstoff auf 50°C erwärmt. Es werden bei einer Rührgeschwindigkeit von 300 Umdrehungen pro Minute 2,3 g Wasser zugegeben, wobei weiter Stickstoff durch die Lösung geleitet wird. Die Polymerisation wird durch Zugabe von 0,14 g Azobis(2,4-Dimethyl-valeronitril) gestartet. Nach einer Reaktionsdauer von 20 Minuten werden noch einmal 20 g Acrylsäure, 2,3 g Wasser und 1,1 g Blockcopolymer zugegeben und nach weiteren 15 Minuten erneut 20 g Acrylsäure, 2,3 g Wasser und 1,1 g Blockcopolymer. Nach einer Gesamtreaktionsdauer von 50 Minuten ist die Polymerisation beendet. Gegebenenfalls können zur Vernetzung danach noch 2,5 g Ethylenglykoldiglycidylether unter gleichzeitiger Erhöhung der Reaktortemperatur auf 60 bis 70°C zugegeben werden. Die Reaktionszeit für die Vernetzung beträgt ca. 2 bis 3 h.

Nach dem Abkühlen auf 20°C wird eine gelblich trübe, violett irisierende Dispersion erhalten. Die Teilchengröße beträgt ca. 170 nm.

## Patentansprüche

1. Poly(meth)acrylsäure-Dispersion mit einem organischen Lösungsmittel als äußerer Phase, wobei die disperse Phase Teilchen einer mittleren Teilchengröße von etwa 20 bis 300 nm bei geringer Polydispersität aufweist, erhältlich durch radikalische Polymerisation von (Meth)acrylsäure und gegebenenfalls bis zu 5 Gew.-% Comonomeren, welche eine Vernetzung der Poly(meth)acrylsäure bewirken, in einem unpolaren organischen Lösungsmittel in Gegenwart von Stabilisatoren, Costabilisatoren und Initiatoren, wobei
(1) der Stabilisator ein Blockcopolymerisat ist, welches aus mindestens einem Block A und mindestens einem Block B besteht, wobei der Block A durch Polymerisation von Vinylgruppen enthaltenden Monomeren gebildet ist und der Block B ein Polyoxyalkylenblock ist,
(2) das Gewichtsverhältnis von Stabilisator zur Summe von (Meth)acrylsäure und gegebenenfalls enthaltenen Comonomeren etwa 1 : 3 bis 1 : 100 beträgt,
(3) der Costabilisator Wasser oder ein Alkandiol mit bis zu 3 Kohlenstoffatomen ist, und
(4) das Gewichtsverhältnis von Costabilisator zur Summe von (Meth)acrylsäure und gegebenenfalls enthaltenen Comonomeren ≥ 1 : 50 und höchstens 1 : 1 beträgt, und
(5) das Gewichtsverhältnis von äußerer zu innerer Phase etwa 100 : 5 bis 100 : 70 beträgt.

2. Poly(meth)acrylsäure-Dispersion nach Anspruch 1, dadurch gekennzeichnet, daß der in ihr enthaltene Stabilisator ein Blockcopolymerisat des Typs A-B oder A-B-A ist.

3. Poly(meth)acrylsäure-Dispersion nach Anspruch 1 oder 2, dadurch gekennzeichnet, daß die Molmasse des Stabilisators etwa 1 000 bis 20 000 beträgt.

4. Poly(meth)acrylsäure-Dispersion nach einem oder mehreren der vorhergehenden Ansprüche, dadurch gekennzeichnet, daß das Verhältnis der anteiligen Molmassen der Blöcke A und B etwa 0,5 : 1 bis 9 : 1 beträgt.

5. Poly(meth)acrylsäure-Dispersion nach einem oder mehreren der vorhergehenden Ansprüche, dadurch gekennzeichnet, daß der Block B aus mindestens 70 Mol-% Oxyethyleneinheiten, Rest Oxyalkyleneinheiten größerer Kohlenstoffzahl besteht.

6. Poly(meth)acrylsäure-Dispersion nach einem oder mehreren der vorhergehenden Ansprüche, dadurch gekennzeichnet, daß das Gewichtsverhältnis von Stabilisator zur Summe von (Meth)acrylsäure und gegebenenfalls enthaltenen Comonomeren etwa 1 : 10 bis 1 : 20 beträgt.

7. Poly(meth)acrylsäure-Dispersion nach einem oder mehreren der vorhergehenden Ansprüche, dadurch gekennzeichnet, daß der Costabilisator Wasser ist und das Gewichtsverhältnis von Wasser zur Summe von (Meth)acrylsäure und gegebenenfalls enthaltenen Comonomeren ≥ 1 : 20 bis ≤ 1 : 5 beträgt.

8. Poly(meth)acrylsäure-Dispersion nach einem oder mehreren der vorhergehenden Ansprüche, dadurch gekennzeichnet, daß die äußere Phase ein aromatisches Lösungsmittel oder Lösungsmittelgemisch mit einem Siedepunkt ≤ 150°C ist.

9. Poly(meth)acrylsäure-Dispersion nach einem oder mehreren der vorhergehenden Ansprüche, dadurch gekennzeichnet, daß die äußere Phase Toluol enthält.

10. Poly(meth)acrylsäure-Dispersion nach einem oder mehreren der vorhergehenden Ansprüche, dadurch gekennzeichnet, daß man als vernetzende Comonomere Divinylbenzol, Ethylenglykol-, Diethylenglykol- oder Butandiol-1,4-di(meth)acrylat einsetzt.

11. Poly(meth)acrlysäure-Dispersion nach einem oder mehreren der vorhergehenden Ansprüche, dadurch gekennzeichnet, daß man die erhaltene Poly(meth)acrylsäure mit einem Diglycidylether nachträglich vernetzt.

12. Poly(meth)acrylsäure-Dispersion nach Patentanspruch 11, dadurch gekennzeichnet, daß man für die nachträgliche Vernetzung Butylenglykoldiglycidylether verwendet.

## Claims

1. Poly(meth)acrylic acid dispersion having an organic solvent as the external phase, the disperse phase comprising particles having an average particle size of about 20 to 300 nm with a low polydispersity, obtainable by free radical polymerization of (meth)acrylic acid and, if appropriate, up to 5% by weight of comonomers, which effect crosslinking of the poly(meth)acrylic acid, in a non-polar organic solvent in the presence of stabilizers, costabilizers and initiators, wherein
(1) the stabilizer is a block copolymer which comprises at least one block A and at least one block B, block A being formed by polymerization of monomers containing vinyl groups and block B being a polyoxyalkylene block,
(2) the weight ratio of stabilizer to the sum of (meth)acrylic acid and any comonomers present is about 1 : 3 to 1 : 100,
(3) the costabilizer is water or an alkanediol having up to 3 carbon atoms and
(4) the weight ratio of costabilizer to the sum of (meth)acrylic acid and any comonomers present is ≥ 1 : 50 and not more than 1 : 1, and
(5) the weight ratio of the external to the internal phase is about 100 : 5 to 100 : 70.

2. Poly(meth)acrylic acid dispersion according to Claim 1, characterized in that the stabilizer present therein is a block copolymer of the type A-B or A-B-A.

3. Poly(meth)acrylic acid dispersion according to Claim 1 or 2, characterized in that the molecular weight of the stabilizer is about 1,000 to 20,000.

4. Poly(meth)acrylic acid dispersion according to one or more of the preceding claims, characterized in that the ratio of the pro rata molecular weights of blocks A and B is about 0.5 : 1 to 9 : 1.

5. Poly(meth)acrylic acid dispersion according to one or more of the preceding claims, characterized in that block B comprises at least 70 mol% of oxyethylene units, the remainder being oxyalkylene units of higher carbon number.

6. Poly(meth)acrylic acid dispersion according to one or more of the preceding claims, characterized in that the weight ratio of stabilizer to the sum of (meth)acrylic acid and any comonomers present is about 1 : 10 to 1 : 20.

7. Poly(meth)acrylic acid dispersion according to one or more of the preceding claims, characterized in that the costabilizer is water and the weight ratio of water to the sum of (meth)acrylic acid and any comonomers present is ≥ 1 : 20 to ≤ 1 : 5.

8. Poly(meth)acrylic acid dispersion according to one or more of the preceding claims, characterized in that the external phase is an aromatic solvent or solvent mixture having a boiling point ≤ 150°C.

9. Poly(meth)acrylic acid dispersion according to one or more of the preceding claims, characterized in that the external phase comprises toluene.

10. Poly(meth)acrylic acid dispersion according to one or more of the preceding claims, characterized in that divinylbenzene or the di(meth)acrylate of ethylene glycol, diethylene glycol or butane-1,4-diol is employed as the crosslinking comonomer.

11. Poly(meth)acrylic acid dispersion according to one or more of the preceding claims, characterized in that the poly(meth)acrylic acid obtained is subsequently crosslinked with a diglycidyl ether.

12. Poly(meth)acrylic acid dispersion according to Patent Claim 11, characterized in that butylene glycol diglycidyl ether is used for the subsequent crosslinking.

## Revendications

1. Dispersion d'acide poly(méth)acrylique avec un solvant organique comme phase externe, dans laquelle la phase dispersée a des particules d'une dimension moyenne d'environ 20 à 300 nm avec une faible polydispersivité, qu'on peut obtenir par polymérisation radicalaire d'acide (méth)acrylique et éventuellement jusqu'à 5 % en poids de comonomères qui provoquent une réticulation de l'acide poly(méth)acrylique, dans un solvant organique non polaire, en présence de stabilisateurs, de co-stabilisateurs et d'initiateurs, dans laquelle :
(1) le stabilisateur est un copolymère séquencé qui est formé d'au moins une séquence A et d'au moins une séquence B, la séquence A étant formée par polymérisation de monomères contenant des groupes vinyliques, et la séquence B étant une séquence de polyoxyalkylène,
(2) le rapport en poids du stabilisateur à la somme de l'acide (méth)acrylique et des comonomères éventuellement contenus est d'environ 1 : 3 à 1 : 100,
(3) le co-stabilisateur est l'eau ou un alcane-diol ayant jusqu'à 3 atomes de carbone, et
(4) le rapport en poids du co-stabilisateur à la somme de l'acide (méth)acrylique et des comonomères éventuellement contenus est ≥ 1 : 50 et d'au plus 1 : 1, et
(5) le rapport en poids de la phase externe à la phase interne est d'environ 100 : 5 à 100 : 70.

2. Dispersion d'acide poly(méth)acrylique selon la revendication 1, caractérisée en ce que le stabilisateur qu'elle contient est un copolymère séquencé du type A-B ou A-B-A.

3. Dispersion d'acide poly(méth)acrylique selon la revendication 1 ou 2, caractérisée en ce que le poids moléculaire du stabilisateur est de 1 000 à 20 000.

4. Dispersion d'acide poly(méth)acrylique selon une ou plusieurs des revendications précédentes, caractérisée en ce que le rapport des poids moléculaires respectifs des séquences A et B est d'environ 0,5 : 1 à 9 : 1.

5. Dispersion d'acide poly(méth)acrylique selon une ou plusieurs des revendications précédentes, caractérisée en ce que la séquence B est formée d'au moins 70 % en moles d'unités oxyéthylène, le reste étant formé d'unités oxyalkylène ayant un nombre plus élevé d'atomes de carbone.

6. Dispersion d'acide poly(méth)acrylique selon une ou plusieurs des revendications précédentes, caractérisée en ce que le rapport en poids du stabilisateur à la somme de l'acide (méth)acrylique et des monomères éventuellement contenus est d'environ 1 : 10 à 1 : 20.

7. Dispersion d'acide poly(méth)acrylique selon une ou plusieurs des revendications précédentes, caractérisée en ce que le co-stabilisateur est formé d'eau, et le rapport de l'eau à la somme de l'acide (méth)acrylique et des monomères éventuellement contenus est de ≥ 1 : 20 à ≤ 1 : 5.

8. Dispersion d'acide poly(méth)acrylique selon une ou plusieurs des revendications précédentes, caractérisée en ce que la phase externe est un solvant aromatique ou un mélange de solvants ayant un point d'ébullition ≤ 150°C.

9. Dispersion d'acide poly(méth)acrylique selon une ou plusieurs des revendications précédentes, caractérisée en ce que la phase externe contient du toluène.

10. Dispersion d'acide poly(méth)acrylique selon une ou plusieurs des revendications précédentes, caractérisée en ce qu'on utilise, comme comonomères de réticulation, le divinylbenzène, le di(méth)acrylate d'éthylèneglycol, le di(méth)acrylate de diéthylèneglycol ou le di(méth)acrylate de butanediol-1,4.

11. Dispersion d'acide poly(méth)acrylique selon une ou plusieurs des revendications précédentes, caractérisée en ce que l'acide poly(méth)acrylique obtenu est réticulé ultérieurement avec un éther diglycidylique.

12. Dispersion d'acide poly(méth)acrylique selon la revendication 11, caractérisée en ce qu'on utilise un éther diglycidylique de butylèneglycol pour la réticulation ultérieure.
